# EUROPÄISCHE PATENTSCHRIFT

(11) **EP 1 220 663 B1**
(45) Veröffentlichungstag und Bekanntmachung des Hinweises auf die Patenterteilung: **16.11.2005**
(21) Anmeldenummer: 00969373.0
(22) Anmeldetag: 30.09.2000
(51) Int. Cl.: A61K 9/70, A61K 7/48, A61K 31/60

(54) **SUPERFIZIELLES THERAPEUTISCHES SYSTEM ZUR TOPISCHEN APPLIKATION VON ACETYLSALICYLSÄURE ZUR BEHANDLUNG VON AKNE-ERKRANKUNGEN**
SUPERFICIAL THERAPEUTIC SYSTEM FOR TOPICALLY APPLYING ACETYLSALICYLIC ACID, FOR THE TREATMENT OF ACNE-RELATED DISEASES
SYSTEME THERAPEUTIQUE SUPERFICIEL PERMETTANT L'APPLICATION TOPIQUE D'ACIDE ACETYLSALICYLIQUE DANS LE BUT DE TRAITER L'ACNE ET LES MALADIES APPARENTEES

(30) Priorität: 13.10.1999 DE 19949252
(43) Veröffentlichungstag der Anmeldung: 10.07.2002
(73) Patentinhaber: LTS LOHMANN Therapie-Systeme AG, 56626 Andernach (DE)
(72) Erfinder: ASMUSSEN, Bodo, 56170 Bendorf (DE); SELZER, Torsten, 56564 Neuwied (DE); HOFFMANN, Hans-Rainer, 56566 Neuwied (DE)
(74) Vertreter: Flaccus, Rolf-Dieter
(86) Internationale Anmeldenummer: PCT/EP2000/009615
(87) Internationale Veröffentlichungsnummer: WO 2001/026636

(56) Entgegenhaltungen:
- WO-A-95/08330
- WO-A-97/48387
- DE-A- 3 413 052
- DE-A- 4 241 128
- DE-A- 19 701 059

## Beschreibung

Die Erfindung betrifft die Vernaendung von Acetylsalicylsäure zur Herstellung eines superfiziellen therapeutischen Systems zur topischen Verabreichung von Acetylsalicylsäure zur Behandlung der Akne und verwandter Hauterkrankungen wie beamsprucht in dem Ansprüchen. Die Erfindung umfaßt ferner Verfahren zur Herstellung solcher System.

Unter der Bezeichnung "Akne" werden verschiedene Talgdrüsenerkrankungen der Haut zusammengefaßt. Die mikroskopisch kleinen Talgdrüsen sind besonders in der Gesichtshaut, der Haut des Schultergürtels, des Rückens, und der Brust besonders zahlreich vertreten. Als charakteristische Krankheitssymptome treten die sogenannten Komedonen (Mitesser) auf. Dabei handelt es sich Talgdrüsen, in denen sich vermehrt Talg angesammelt hat. Die Ursache hierfür ist zum einen eine überhöhte Talgabsonderung, beispielsweise aufgrund hormoneller Einflüsse, und zum anderen eine verstärkte Verhomung, durch welche der Talgabfluß aus den Drüsen behindert wird. In der Folge können sich die Komedonen entzünden, und es kommt zur Bildung von Papeln und Pusteln, welche vielfach als "Pickel" bezeichnet werden. Für die Entzündungsreaktionen werden vor allem die auf der Hautoberfläche befindlichen Bakterien verantwortlich gemacht.

Für die therapeutische Behandlung der Akne stehen derzeit verschiedene medikamentöse Methoden zur Verfügung, die jedoch allesamt mit gewissen Nachteilen behaftet sind. In den meisten Fällen wird Akne mittels Salben, Gelen, Emulsionen, Lotionen oder Lösungen, also topisch behandelt. Als Wirkstoffe werden vor allem Antibiotika (insbesondere Erythromycine, Tetracycline), Keratolytica, ätzende Pharmaka, Anti-Androgene, Retinoide und Benzoylperoxid eingesetzt.

Bei der Anwendung der genannten Arzneiformen kann sich allerdings die unkontrollierte Abgabe des Wirkstoffes vom Träger in die Haut als nachteilig erweisen. Zudem werden diese Arzneiformen aus praktischen Gründen meist großflächig appliziert, so daß neben den von Akne betroffenen Hautbereichen auch gesunde Hautareale mit der wirkstoffhaltigen Arzneizubereitung in Berührung kommen. Dies kann sich als nachteilig erweisen, nämlich wenn die Wirkstoffe die Eigenschaft haben, gesunde Haut zu reizen. Dies ist z. B. bei Salicylsäure, Benzoylperoxid und Schwefel der Fall.

Die beschriebenen Nachteile können vermieden werden, wenn als Darreichungsform ein superfizielles therapeutisches System (STS) in Form eines wirkstoffhaltigen Pflasters verwendet wird. Dieses bietet den Vorteil, daß es gezielt auf den zu behandelnden Hautbereich appliziert werden kann. Darüber hinaus können bei der Verabreichung mittels pflasterförmiger STS konstante und kontrollierte Abgaberaten erzielt werden. Der Einsatz von wirkstoffhaltigen Pflastern zur Behandlung der Akne ist in der US-PS 5 409 917 und in der WO 97/48 387 beschrieben.

Die Eignung der Acetylsalicylsäure zur Akne-Therapie beruht vor allem auf ihrer entzündungshemmenden und schmerzstillenden Wirkung, welche auf eine Hemmung des Prostaglandin-Synthesewegs zurückzuführen ist. Die therapeutische Wirksamkeit der Acetylsalicylsäure bei der Behandlung von Akne ist beispielsweise aus der US-PS 4 665 063 bekannt. Die Permeationsfähigkeit von Acetylsalicylsäure durch die menschliche Haut, und damit ihre grundsätzliche Eignung als Bestandteil von wirkstoffhaltigen Hautpflastern ist bekannt (Journal of Pharmaceutical Science 176 (1987), pp. 451-454).

WO 95 08330 A offenbart ein Acetylsalicylsäure enthaltendes transdermales therapeutisches System mit einer oder mehreren wirkstoffhaltigen Matrixschichten. Mindestens eine der Matrixschichten enthält Acetylsalicylsäure zum überwiegenden Teil in kristalliner Form. Acetylsalicylsäure wird als Antirheumatikum, Antipyretikum, Analgetikum und Thrombozyten-Aggregationshemmer beschrieben.

DE 34 13 052 A betrifft eine wasserfreie pharmazeutische Zubereitung, beispielsweise eine Salbe oder Lotion, die als aktiven Bestandteil Acetylsalicylsäure zusammen mit einer geeigneten Basis (z. B. Paraffin, Lanolin) enthält. Die Zubereitung ist für die Behandlung von Hautkrankheiten wie z. B. Akne geeignet.

WO 97 48387 A betrifft ein wirkstoffhaltiges Hautpflaster zur topischen Verabreichung einer Anti-Akne-Formulierung, die mindestens zwei verschiedene Wirkstoffe enthält. Als Wirkstoff wird u. a. Salicylsäure, nicht aber ASS in Betracht gezogen. Salicylsäure wird aufgrund ihrer keratolytischen Wirkung verwendet.

DE 197 01 059 A offenbart ein transdermales therapeutisches System, bei dem ASS in den Grundpolymeren gelöst oder dispergiert vorliegt. Dieses System ist zur systemischen, nicht topischen Verabreichung des Wirkstoffs ASS bestimmt. Acetylsalicylsäure wird als Antirheumatikum, Antipyretikum, Analgetikum und Thrombozyten-Aggregationshemmer beschrieben.

DE 42 41 128 A beschreibt ein Verfahren zur Herstellung eines ASS-haltigen transdermalen Verabreichungssystems, wobei ASS in fester Form oder in Form einer Lösung oder Dispersion in das System eingebracht wird. Dieses System ist zur systemischen, nicht topischen Verabreichung des Wirkstoffs ASS bestimmt, und zwar für die antithrombotische Therapie.

Eine Hauptschwierigkeit bei der Herstellung von acetylsalicylsäurehaltigen Hautpflastern, wie sie aus dem Stand der Technik bekannt sind, besteht darin, daß einerseits eine hohe Abgabegeschwindigkeit erzielt werden muß und gleichzeitig eine ausreichende pharmakologische Stabilität gewährleistet werden muß.

Die der Erfindung zugrundeliegende Aufgabe bestand darin, eine topische Darreichungsform für Acetylsalicylsäure zu schaffen, welche für die therapeutische

Behandlung von Akne und ähnlichen Krankheitsbildern verwendet werden kann, und welche sowohl eine ausreichende pharmazeutische Stabilität wie auch eine - unter den gegebenen Umständen - maximale Abgabegeschwindigkeit an die Haut aufweist. Weiter bestand die Aufgabe darin, ein Verfahren aufzuzeigen, welches die Herstellung derartiger Applikationssyteme ermöglicht.

Überraschenderweise wird die Aufgabe durch die Vernaendung gemäß Anspruch 1 und durch die in den Unteransprüchen 2 bis 11 beschriebenen bevorzugten Ausführungsformen gelöst. Die Ansprüche 12 bis 14 geben das erfindungsgemäße Herstellungsverfahren an.

Das erfindungsgemäße superfizielle therapeutische System zur topischen Behandlung der Akne weist einen mehrschichtigen Aufbau auf, der eine Rückschicht, mindestens eine wirkstoffhaltige Matrixschicht und eine ablösbare Schutzfolie umfaßt. Die Rückschicht ist wirkstoff- und feuchtigkeitsundurchlässig. Das System ist dadurch gekennzeichnet, daß der Wirkstoff Acetylsalicylsäure mindestens in einer der Matrixschichten zu einem Teil in suspendierter Form bzw. im festen Aggregatszustand (kristallin oder amorph) vorliegt. Dieser Anteil beträgt mindestens 30 %, noch besser mindestens 50 %, bezogen auf die gesamte Masse der enthaltenen Acetylsalicylsäure.

Durch die Anwesenheit einer suspendierten, d. h. kristallinen und / oder amorphen Wirkstoffphase, zusammen mit gelöstem Wirkstoff, wird eine Stabilisierung des Wirkstoffs bewirkt und es werden unkontrollierte Rekristallisationen verhindert. Gleichzeitig führt der suspendierte Wirkstoffanteil zu einer - unter den gegebenen Umständen - maximalen Abgabegeschwindigkeit von Wirkstoff an die Haut.

Besonders gute Erfolge werden verzeichnet, wenn der Wirkstoff Acetylsalicylsäure mindestens in einer der Matrixschichten zum überwiegenden Teil in suspendierter Form vorliegt, bevorzugt in einem Anteil von mindestens 50 %, bezogen auf die Gesamtmasse der Acetylsalicylsäure.
Jedoch kann es schon ausreichend sein, nur etwa 10 % oder sogar nur 1 % (bezogen auf die Masse) kristalline oder amorphe Acetylsalicylsäure in stabiler Modifikation in einer gesättigten Lösung von Acetylsalicylsäure - und pharmazeutischen Hilfsstoffen der Matrix - zu dispergieren, und anschließend auf einer Rückschicht oder anderen Trägerfolie zu trocknen.

Um die erfindungsgemäßen Wirkungen zu erzielen, muß während der Herstellung darauf geachtet werden, daß die Wirkstoffpartikel niemals völlig gelöst werden, so daß immer zumindest ein Teil des Wirkstoffs in fester Form verbleibt. Ferner ist auf eine möglichst homogene Verteilung der Wirkstoffpartikel in der Wirkstoffmatrix zu achten.
Die Größe der Acetylsalicylsäurepartikel ist für die erfindungsgemäße Funktion von geringerer Bedeutung, allerdings werden zur Erzielung eines homogeneren optischen Eindrucks kleinere Wirkstoffpartikel mit Durchmessern unterhalb von 50 bis 100 µm bevorzugt. Werden größere Wirkstoffpartikel (über 300 µm Durchmesser) in Polymermatrices mit sehr schlechten Lösungseigenschaften, z. B. basierend auf Polyisobutylen, verwendet, so kann dies dazu führen, daß die Freisetzungskontrolle des Wirkstoffs durch die Haut erfolgt. Dies hat den Nachteil, daß die dabei erzielten Flußraten unterhalb des theoretisch erreichbaren Maximalwerts bleiben.

Vorzugsweise verwendet man zur Herstellung der wirkstoffhaltigen Schichten wasserfreie kristalline Acetylsalicylsäure, wobei man von derjenigen Modifikation der Acetylsalicylsäure ausgeht, welche oberhalb von 132 °C schmilzt und als die thermodynamisch stabilste Form gilt.

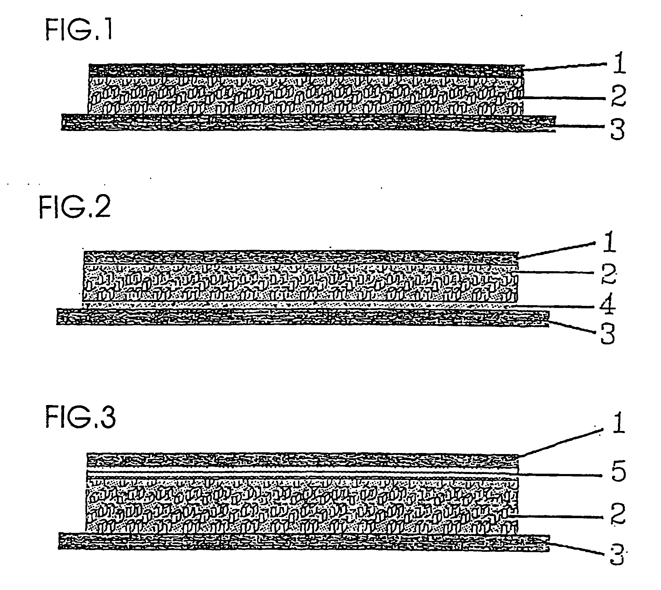

In einer bevorzugten Ausführungsform (siehe Fig. 1) besteht das superfizielle therapeutische System aus einer Rückschicht (1), einer einschichtigen Matrix (2) sowie einervor der Anwendung zu entfernenden ablösbaren Schutzfolie (3). Die wirkstoffhaltige Matrix weist dabei haftklebende Eigenschaften auf. Darüber hinaus kann der Schichtaufbau, insbesondere die Art und Anzahl der Matrixschichten, abhängig von den jeweiligen Erfordernissen unterschiedlich ausgestaltet werden.

In einer weiteren bevorzugten Ausführungsform (siehe Fig. 2) besteht die Matrixschicht des superfziellen therapeutischen Systems aus zwei wirkstoffhaltigen Schichten (2) und (4), welche beide Acetylsalicylsäure zum Teil in suspendierter Form enthalten. Dabei ist lediglich die hautseitig gelegene Matrixschicht (4) haftklebend ausgerüstet. Diese enthält in diesem Fall nur einen geringen Anteil von suspendierter Acetylsalicylsäure, d. h. kleinere Wirkstoffpartikel und in geringerer Häufigkeit, um eine bessere Haftung dieser Matrixschicht (4) auf der Haut zu erreichen. Ein vollständiger Verzicht auf Wirkstoffpartikei in der hautnahen Matrixschicht (4) ist allerdings nicht ratsam, da sonst eine zu breite Diffusionsgrenzschicht entstehen könnte, welche den Wirkstofffluß in die Haut beeinträchtigen würde.

Der Anteil von Acetylsalicylsäure am Matrixmaterial beträgt vorzugsweise 15 bis 60 %, bezogen auf die Gesamtmasse der Matrix, wobei insbesondere Werte zwischen 35 bis 50 % bevorzugt werden. Besonders bei gut lösenden Matrixpolymeren, wie z. B. Acrylsäureestercopolymeren, ist eine Überschreitung der Sättigungslöslichkeit um den Faktor 2 oder sogar etwa 5 bis 10 nur mit einer solchen sehr hohen Beladung des Systems mit Wirkstoff zu erreichen.

Als Grundmaterial für die Matrixschicht (2) und die ebenfalls wirkstoffhaltige Klebschicht (4) werden vorzugsweise Acrylsäureester-Copolymere eingesetzt. Daneben können beispielsweise auch Mischungen aus Kautschuken und Harzen, ferner Polyvinylacetat, Silikonpolymere, Styrol-Dien-Copolymere, Mischungen von Polyisobutylen mit thermoplastischen Harzen, und andere für die Haut unbedenkliche Materialien eingesetzt werden. Um die kohäsiven Eigenschaften zu verbessern, können bis zu 40 % (bezogen auf die Matrixmasse) Füllstoffe zugesetzt werden, wie z. B. Titandioxid, Zinkoxid, Kreide, Aktivkohle oder feinverteiltes Siliciumdioxid. Die erfindungsgemäße Wirkung wird durch derartige Zusätze nicht beeinträchtigt.

Als Rückschicht (1) der superfiziellen therapeutischen Systeme eignen sich vor allem Polyester, die sich durch besondere Festigkeit und Diffusionsfestigkeit auszeichnen, darüber hinaus aber nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid ähnlicher, dem Fachmann bekannter Stoffe. Zur Verbesserung des äußeren Erscheinungsbilds kann die Rückschicht auch auf der Außenseite hautfarben lackiert werden oder auf andere Weise behandelt werden.
Abhängig von der Festigkeit und Durchlässigkeit des gewählten Materials beträgt die Dicke der folienartigen Rückschicht gewöhnlich 8 bis 80 µm. Sie kann für spezielle Zwecke jedoch auch dicker oder dünner als diese Werte eingestellt werden.

Die wiederablösbare, vor Anwendung des Pflasters zu entfernende Schutzschicht (3) besteht vorzugsweise aus Polyestermaterial, aber auch beliebige andere hautverträgliche Kunststoffe können verwendet werden, wie z. B. Polyvinylchlorid, Ethylenvinylacetat, Vinylacetat, Polyethylen, Polypropylen oder Cellulosederivate. Im Einzelfall kann eine Bedampfung mit Metallen oder anderen diffusionssperrenden Zusatzstoffen wie Siliciumdioxid, Aluminiumoxid o. ä vorgenommen werden. In jedem Fall ist zur Seite der klebenden Matrix hin eine Oberflächenbeschichtung mit dehäsiven Materialien erforderlich, beispielsweise mit Silikonen oder fluorhaltigen Kunststoffen, damit der Verbund leicht ablösbar bleibt.
Die Dicke der folienartigen wiederablösbaren Schutzschicht beträgt gewöhnlich 40 bis 200 µm; sie kann für spezielle Zwecke auch dicker oder dünner eingestellt werden.

Es sind ferner Ausführungsformen der Erfindung vorgesehen, bei welchen die Matrixschicht(en) (2, 5) oder die Klebschicht (4) Zusatzstoffe enthält. Dazu zählen insbesondere Stoffe aus der Gruppe der Absorptions- oder Permeationsbeschleuniger, der Weichmacher und Klebrigmacher.
Vorzugsweise werden flüssige Zusatzstoffe zugesetzt, wie z. B. 2-Pyrrolidon, Benzylalkohol, Butanol und andere kurzkettige Alkohole, Triglyceride, Cholesterol, Cineol, A-Tocopherol, Diethylenglykol, Diethylenglykolmonoethylether, Diisopropyladipat, Dimethyldecylphosphoxid, Dimethylisosorbid, Dimethyllauroylamid, Dimethylsulfoxid, Dodecylsutfoxid, Essigsäure, Ethylacetat, und andere aliphatische und aromatische Ester, Ethylenglycol, Ethylenglycolmonolaurat und andere Ester und Ether von Ethylenglycol oder Propylenglycol, 2-Octyldodecanol, dünnflüssiges Paraffin, Glycerin, Glycerinmonooleat, Glycerinmonostearat, hydriertes Rizinusöl, Isopropylmyristat, Isopropylpalmitat, Laurinsäurediethanolamid, Menthol oder andere flüchtige Terpenderivate, Terpenderivate oder Terpenderivate enthaltende Gemische aus natürlichen etherischen Ölen, Methylbenzoat, Methyloctylsulfoxid, Mono- oder Diethylacetamid, N,N-Diethyl-m-toluamid, N-Methylpyrrolidon, Octanol-1 und andere flüchtige mittelkettige Alkohole, Octansäure und andere mittelkettige aliphatische Carbonsäuren, Oleylalkohol, Olivenöl, Ölsäure, Ölsäureoleylester, Phenylethanol, Propylenglykol, Rizinolsäure, Triacetin, aber auch Mischungen solcher Stoffe, wie z. B. ölsäure/Propylenglykol.

Bei der Verwendung von Zusatzstoffen in der wirkstoffhaltigen Matrix ist jedoch im Einzelfall die Reaktionsfähigkeit der Acetylsalicylsäure mit Estern und Säuren sowie Alkoholen zu berücksichtigen, wodurch der Einsatz dieser Zusatzstoffe limitiert wird. Die vorliegende Erfindung grenzt jedoch mögliche chemische Reaktionen durch Kompartimentierung weitestmöglich ein, so daß auch in dieser Hinsicht ein Stabilitätsvorteil erreicht wird.

Diese Kompartimentierung kann mit einer weiteren bevorzugten Ausführungsform der Erfindung (Fig. 3) bewirkt werden, bei welcher ebenfalls zwei wirkstoffhaltige Matrixschichten vorliegen, die Acetylsalicylsäure in suspendierter Form enthalten und bei der Herstellung aufeinander laminiert werden. Dabei enthält eine der beiden Matrixschichten (5) einen leicht flüchtigen Inhaltsstoff, beispielsweise ein Lösemittel, welches auch Hilfsstoffe wie z. B. Absorptionsbeschleuniger oder Weichmacher aufweisen kann. Auf diese Weise kann nach erfolgter Wanderung der flüchtigen Komponente in die andere Matrixschicht (2 ein ausreichend scherstabiles System erhalten werden, welches flüchtige Zusatzstoffe enthält.

Für bestimmte Anwendungen kann es vorteilhaft oder notwendig sein, daß die erfindungsgemäßen acetylsalicylsäurehaltigen superfiziellen therapeutischen Systeme zusätzlich mindestens einen weiteren Wirkstoff enthalten. Diese Wirkstoffe können vorzugsweise ausgewählt werden aus der Gruppe, welche Antibiotika, Keratolytika, ätzende Verbindungen, Anti-Androgene, Retinoide, Benzoylperoxid und Schwefel umfaßt.

Die Herstellung der superfiziellen therapeutischen Systeme erfolgt gemäß dem erfindungsgemäßen Verfahren auf die Weise, daß die vorliegende Acetylsalicylsäure in einer Suspension bzw. Lösung oder in einer Schmelze des Matrix-Grundmaterials auf einem folienförmigen, dehäsiv ausgerüsteten Trägermaterial - z. b. einer wiederablösbaren Schutzschicht (3) - in einer Schicht aufgebracht und getrocknet wird. Danach wird die getrocknete Schicht mit einer wirkstoff- und feuchtigkeitsundurchlässigen Rückschicht (1) bedeckt und das Substrat durch Konturstanzung bzw. Folienschnitt vereinzelt.

Um die wirkstoffhaltigen superfiziellen therapeutischen Systeme gegen physikalische Alterungseinflüsse zu stabilisieren, ist es vorteilhaft, diese nach erfolgter Vereinzelung in gas- und feuchtigkeitsdicht versiegelte Packmittel zu verpacken. Vorzugsweise ist das Packmittel wasseraufnehmend ausgerüstet, so daß es dem System Feuchtigkeit zu entziehen vermag. Ferner können auch feuchtigkeitsabsorbierende Körper in das Packmittel eingelegt werden.

Vorzugsweise wird zumindest eine Teilmange des im Herstellungsprogeß eingesetzten Wirkstoffs während der gesamten Zeitdaner der Fertigung in suspendiertem Zustand gehalten.

Der Aufbau der acetylsalicylsäurehaltigen superfiziellen therapeutischen Systeme (STS) ist in den FIG. 1-3 schematisch dargestellt.

FIG. 1 zeigt ein erfindungsgemäßes STS mit einem dreischichtigen Aufbau, bestehend aus einer wirkstoffundurchlässigen Rückschicht (1), einer wirkstoffhaltigen Matrixschicht (2) und einer ablösbaren Schutzfolie (3).

FIG. 2 zeigt eine Ausführungsform eines STS mit zwei wirkstoffhaltigen Matrixschichten (2) und (4), wobei (4) die hautnahe Matrixschicht darstellt. (1) und (3) bezeichnen die Rückschicht bzw. die ablösbare Schutzfolie. Nur die hautnahe Matrixschicht (4) ist haftklebend ausgebildet. Sie enthält kleinere und weniger Acetylsalicylsäure-Partikel als die Matrixschicht (2).

FIG. 3 zeigt eine weitere Ausführungsform eines erfindungsgemäßen STS. Die hautferne Matrixschicht (5) enthält ebenfalls wie die Matrixschicht (2) Acetylsalicylsäure. Die hautferne Matrixschicht (5) besteht aus einer Lösung der Matrixbestandteile in einem im STS verbleibenden Lösungsmittel. (1) und (3) bezeichnen die Rückschicht bzw. die ablösbare Schutzfolie.

## Patentansprüche

1. Verwendung von Acetylsalicylsäure zur Herstellung eines superfiziellen therapeutischen Systems mit einer wirkstoff- und feuchtigkeitsundurchlässigen Rückschicht, einer oder mehrerer wirkstoffhaltiger Matrixschichten und einer ablösbaren Schutzschicht, bei dem der Wirkstoff Acetylsalicylsäure mindestens in einer der Matrixschichten des superfiziellen therapeutischen Systems teils in fester, suspendierter Form und teils in gelöster Form vorliegt, und der Anteil der in fester Form vorliegenden Acetylsalicylsäure, bezogen auf die gesamte Masse der enthaltenen Acetylsalicylsäure, mindestens 30 % beträgt, für die Behandlung von Akne und/ oder verwandter Hauterkrankungen durch topische Verabreichung von Acteylsalicylsäure.

2. Verwendung von Acetylsalicylsäure nach Anspruch 1, **dadurch gekennzeichnet, daß** der Wirkstoff Acetylsalicylsäure mindestens in einer der Matrixschichten zum Teil in kristalliner Form vorliegt.

3. Verwendung von Acetylsalicylsäure nach Anspruch 1 oder 2, **dadurch gekennzeichnet, daß** der Wirkstoff Acetylsalicylsäure mindestens in einer der Matrixschichten zum Teil in amorpher Form vorliegt.

4. Verwendung von Acetylsalicylsäure nach Anspruch 1 oder 2, **dadurch gekennzeichnet, daß** die zur Herstellung der wirkstoffhaltigen Schichten verwendete Acetylsalicylsäure als stabile, wasserfreie, oberhalb von 132 °C schmelzende Modifikation in kristalliner Form vorliegt.

5. Verwendung von Acetylsalicylsäure nach einem der vorhergehenden Ansprüche, **dadurch gekennzeichnet, daß** das System eine einschichtige wirkstoffhaltige Matrix mit haftklebenden Eigenschaften aufweist.

6. Verwendung von Acetylsalicylsäure nach einem oder mehreren der vorangehenden Ansprüche, **dadurch gekennzeichnet, daß** das System zwei Matrixschichten aufweist, welche beide Acetylsalicylsäure zum Teil in suspendierter, fester Form enthalten, wobei die hautseitig gelegene Matrixschicht haftklebend ausgerüstet ist und einen geringeren Anteil von kristalliner oder amorpher Acetylsalicylsäure enthält als die andere Matrixschicht.

7. Verwendung von Acetylsalicylsäure nach einem oder mehreren der vorangehenden Ansprüche, **dadurch gekennzeichnet, daß** das System zwei Matrixschichten aufweist, welche beide Acetylsalicylsäure zum Teil in suspendierter, fester Form enthalten, wobei die Matrixbestandteile, einschließlich Zusatzstoffe, der hautfernen Matrix in einem Lösemittel gelöst vorliegen, welches vorzugsweise leicht flüchtig ist.

8. Verwendung von Acetylsalicylsäure nach einem oder mehreren der vorhergehenden Ansprüche, **dadurch gekennzeichnet, daß** der Anteil von Acetylsalicylsäure am Matrixmaterial 15 bis 60 % , vorzugsweise 35 bis 50 % beträgt, bezogen auf die Gesamtmasse der Matrix.

9. Verwendung von Acetylsalicylsäure nach einem oder mehreren der vorhergehenden Ansprüche, **dadurch gekennzeichnet, daß** die Matrixschichten und die Klebschicht als Grundmaterial ein Acrylsäureester-Copolymer enthalten.

10. Verwendung von Acetylsalicylsäure nach einem der vorhergehenden Ansprüche, **dadurch gekennzeichnet, daß** die Matrixschicht(en) oder die Klebschicht Zusatzstoffe enthält, ausgewählt aus der Absorptions- oder Permeationsbeschleuniger, Weichmacher und Klebrigmacher enthaltenden Gruppe.

11. Verwendung von Acetylsalicylsäure nach einem der vorhergehenden Ansprüche, **dadurch gekennzeichnet, daß** das System zusätzlich weitere Wirkstoffe enthält, vorzugsweise ausgewählt aus der Gruppe, welche Antibiotika, Keratolytika, ätzende Verbindungen, Anti-Androgene, Retinoide, Benzoylperoxid und Schwefel umfaßt.

12. Verfahren zur Herstellung eines superfiziellen therapeutischen Systems nach einem oder mehreren der Ansprüche 1 bis 11, **dadurch gekennzeichnet, daß** teils gelöst und teils suspendiert und in amorpher Form vorliegende Acetylsalicylsäure-Partikel auf einem folienförmigen, dehäsiv ausgerüsteten Trägermaterial in einer Schicht aufgebracht und getrocknet werden, und danach die getrocknete Schicht mit einer wirkstoff- und feuchtigkeitsundurchlässigen Rückschicht bedeckt und das Substrat durch Konturstanzung bzw. Folienschnitt vereinzelt wird.

13. Verfahren nach Anspruch 12, **dadurch gekennzeichnet, daß** die superfiziellen therapeutischen Systeme nach Vereinzelung in gas- und feuchtigkeitsdicht versiegelte Packmittel verpackt werden, und daß das Packmittel durch wasseraufnehmende Ausrüstung dem System Feuchtigkeit entzieht.

14. Verfahren nach Anspruch 12 oder 13, **dadurch gekennzeichnet, daß** zumindest eine Teilmenge des im Herstellungsprozeß eingesetzten Wirkstoffs während der gesamten Zeitdauer der Fertigung in suspendiertem Zustand gehalten wird.

## Claims

1. The use of acetylsalicylic acid for the production of a superficial therapeutic system, having an active substance-impermeable and moisture-impermeable back layer, one or more active substance-containing matrix layers, and a separable protective layer, wherein the active substance acetylsalicylic acid is present in at least one of the matrix layers of the superficial therapeutic system partially in solid, suspended form, and partially in dissolved form, and wherein the portion of the acetylsalicylic acid present in solid form, relative to the overall mass of the acetylsalicylic acid contained, amounts to at least 30 %, for the treatment of acne and/or related skin diseases by topical administration of acetylsalicylic acid.

2. The use of acetylsalicylic acid according to claim 1, **characterized in that** the active substance acetylsalicylic acid is present in at least one of the matrix layers partly in crystalline form.

3. The use of acetylsalicylic acid according to claim 1 or 2, **characterised in that** the active substance acetylsalicylic acid is present in at least one of the matrix layers partly in amorphous form.

4. The use of acetylsalicylic acid according to claim 1 or 2, **characterised in that** the acetylsalicylic acid used for the production of the active substance-containing layers is present as a stable, water-free modification in crystalline form melting above 132°C.

5. The use of acetylsalicylic acid according to one of the preceding claims, **characterised in that** the system has a single-layer active substance-containing matrix with pressure-sensitive adhesive features.

6. The use of acetylsalicylic acid according to one or more of the preceding claims, **characterised in that** the system has two matrix layers which both contain acetylsalicylic acid partly in suspended, solid form, the matrix layer adjacent to the skin being rendered pressure-sensitive adhesive and containing a smaller portion of crystalline or amorphous acetylsalicylic acid than the other matrix layer.

7. The use of acetylsalicylic acid according to one or more of the preceding claims, **characterised in that** the system has two matrix layers, which both contain acetylsalicylic acid partly in suspended, solid form, the matrix components, including additives, of the matrix more distant from the skin being present dissolved in a solvent which is preferably readily volatile.

8. The use of acetylsalicylic acid according to one or more of the preceding claims, **characterised in that** the portion of acetylsalicylic acid in the matrix material is 15 to 60%, preferably 35 to 50%, relative to the total mass of the matrix.

9. The use of acetylsalicylic acid according to one or more of the preceding claims, **characterised in that** the matrix layers and the adhesive layer contain an acrylic-acid ester copolymer as base material.

10. The use of acetylsalicylic acid according to one of the preceding claims, **characterised in that** the matrix layer(s) or the adhesive layer contains additives chosen from the group containing absorption or permeation accelerators, plasticizers and tackifiers.

11. The use of acetylsalicylic acid according to one of the preceding claims, **characterised in that** the system moreover contains other active substances, preferably chosen from the group comprising antibiotics, keratolytics, caustic compounds, anti-androgens, retinoids, benzoyl peroxide and sulphur.

12. Process for the production of a superficial therapeutic system according to one or more of the claims 1 to 11, **characterised in that** acetylsalicylic acid particles, which are in part present in dissolved, in suspended and in amorphous form, are applied in a layer onto a film-shaped, dehesively equipped carrier material and dried, and that afterwards the dried layer is covered with an active substance- and moisture-impermeable back layer and the substrate is isolated by contour-punching or film-cutting.

13. Process according to claim 12, **characterised in that** the superficial therapeutic systems are packed into gas- and moisture-proof sealed packages after their isolation and that the package absorbs moisture from the system by means of water-absorbing equipment.

14. Process according to claim 12 or 13, **characterised in that** at least a portion of the active substance used in the production process is kept in a suspended state during the entire period of production.

## Revendications

1. Utilisation d'acide acétylsalicylique pour la préparation d'un système de traitement thérapeutique superficiel, comprenant une couche dorsale imperméable au principe actif et à l'humidité, une ou plusieurs couches matricielles contenant le principe actif, et une couche de protection détachable, le principe actif acide acétylsalicylique étant contenu dans au moins une des couches matricielles du système de traitement thérapeutique superficiel en partie sous forme solide, en suspension, et en partie sous forme dissoute, et la partie de l'acide acétylsalicylique qui est sous forme solide étant d'au moins 30 % par rapport à la masse totale de l'acide acétylsalicylique contenu, pour le traitement de l'acné et/ou et des affections cutanées apparentées, par administration topique d'acide acétylsalicylique.

2. Utilisation d'acide acétylsalicylique selon la revendication 1, **caractérisée en ce que** le principe actif acide acétylsalicylique est contenu dans au moins une des couches matricielles en partie sous forme cristalline.

3. Utilisation d'acide acétylsalicylique selon la revendication 1 ou 2, **caractérisée en ce que** le principe actif acide acétylsalicylique est contenu dans au moins une des couches matricielles en partie sous forme amorphe.

4. Utilisation d'acide acétylsalicylique selon la revendication 1 ou 2, **caractérisée en ce que** l'acide acétylsalicylique utilisé pour la préparation des couches contenant le principe actif est présent en tant que modification, stable, anhydre, ayant un point de fusion supérieur à 132°C, sous forme cristalline.

5. Utilisation d'acide acétylsalicylique selon l'une des revendications précédentes, **caractérisée en ce que** le système présente une matrice monocouche contenant le principe actif, ayant des propriétés adhésives.

6. Utilisation d'acide acétylsalicylique selon une ou plusieurs des revendications précédentes, **caractérisée en ce que** le système présente deux couches matricielles qui contiennent, toutes deux, l'acide acétylsalicylique en partie sous forme solide, en suspension, la couche matricielle tournée vers la peau étant adhésive et contenant une proportion d'acide acétylsalicylique cristalline ou amorphe inférieure à celle de l'autre couche matricielle.

7. Utilisation d'acide acétylsalicylique selon une ou plusieurs des revendications précédentes, **caractérisée en ce que** le système présente deux couches matricielles qui contiennent, toutes deux, l'acide acétylsalicylique en partie sous forme solide, en suspension, les constituants de la matrice éloignée de la peau, y compris les additifs, étant présents sous forme dissoute dans un solvant, ce dernier étant de préférence volatil.

8. Utilisation d'acide acétylsalicylique selon une ou plusieurs des revendications précédentes, **caractérisée en ce que** la proportion d'acide acétylsalicylique dans la substance matricielle est de 15 à 60 %, de préférence de 35 à 50 % par rapport à la masse totale de la matrice.

9. Utilisation d'acide acétylsalicylique selon une ou plusieurs des revendications précédentes, **caractérisée en ce que** les couches matricielles et la couche adhésive contiennent, en tant que matériau de base, un copolymère d'ester acrylique.

10. Utilisation d'acide acétylsalicylique selon l'une quelconque des revendications précédentes, **caractérisée en ce que** la ou les couches matricielles ou la couche adhésive contiennent des additifs choisis dans le groupe comprenant des accélérateurs d'absorption ou de perméation, des émolliants et des substances adhésives.

11. Utilisation d'acide acétylsalicylique selon l'une quelconque des revendications précédentes, **caractérisée en ce que** le système contient en outre d'autres principes actifs, choisis de préférence dans le groupe qui englobe des antibiotiques, des composés kératolytiques, des composés cautérisants, des anti-androgènes, des rétinoïdes, du peroxyde de benzoyle et du soufre.

12. Procédé de préparation d'un système de traitement thérapeutique superficiel selon une ou plusieurs des revendications 1 à 11, **caractérisé en ce que** les particules d'acide acétylsalicylique, qui sont en partie sous forme dissoute et en suspension et sous forme amorphe, sont appliquées puis séchées dans une couche sur un matériau de support en forme de film déhésif, et **en ce qu'**ensuite, la couche séchée est recouverte avec une couche dorsale imperméable au principe actif et à l'humidité, et que le substrat est divisé par découpage suivant un contour ou par découpage du film.

13. Procédé selon la revendication 12, **caractérisé en ce que** les systèmes de traitement thérapeutique superficiels sont conditionnés, après division, dans un moyen de conditionnement scellé de façon imperméable aux gaz et à l'humidité, et **en ce que** ce moyen de conditionnement a une action déshydratante grâce à une garniture absorbant l'eau.

14. Procédé selon la revendication 12 ou 13, **caractérisé en ce qu'**au moins une fraction du principe actif mis en oeuvre dans le procédé de préparation est maintenue en suspension pendant toute la durée de la finition.
